# EUROPEAN PATENT SPECIFICATION

(11) **EP 3 325 480 B1**
(45) Date of publication and mention of the grant of the patent: **22.04.2020**
(21) Application number: 15794055.2
(22) Date of filing: 13.10.2015
(51) Int. Cl.: C07D 487/02, A61K 31/52, A61P 25/00

(54) **5-SUBSTITUTED-7-[4-(SUBSTITUTED) BENZYL]AMINO-3-ISOPROPYLPYRAZOLO (4,3-D) PYRIMIDINES AS MEDICAMENTS**
5-SUBSTITUTIERTE-7-[4-(SUBSTITUTIERTE) BENZYL]AMINO-3-ISOPROPYLPYRAZOLO (4,3-D) PYRIMIDINE ALS MEDIKAMENTE
UTILISATION DE PYRIMIDINES 5-SUBSTITUÉES-7-[4-(SUBSTITUÉE)BENZYL]AMINO-3-ISOPROPYLPYRAZOLO(4,3-D) EN TANT QUE MÉDICAMENTS

(30) Priority: 20.07.2015 CZ 20150509
(43) Date of publication of application: 30.05.2018
(73) Proprietor: Ustav experimentalni botaniky AV CR, v.v.i., 165 02 Praha 6 (CZ); Univerzita Palackeho v Olomouci, 77147 Olomouc (CZ)
(72) Inventor: KRYSTOF, Vladimir, 77900 Olomouc (CZ); VYMETALOVA, Ladislava, 56944 Jaromerice (CZ); HAVLICEK, Libor, 14104 Praha 4 (CZ); STURC, Antonin, 14300 Praha 12 (CZ); JORDA, Radek, 77900 Olomouc (CZ); POSPISIL, Tomas, 77900 Olomouc (CZ); STRNAD, Miroslav, 77900 Olomouc (CZ)
(74) Representative: Hartvichova, Katerina
(86) International application number: PCT/CZ2015/050007
(87) International publication number: WO 2017/012599

(56) References cited:
- WO-A1-2007/134828
- WO-A1-2010/139288
- SROKA IRENE M ET AL: "A novel roscovitine derivative potently induces G1-phase arrest in platelet-derived growth factor-BB-activated vascular smooth muscle cells", MOLECULAR PHARMACOLOGY, AMERICAN SOCIETY FOR PHARMACOLOGY AND EXPERIMENTAL THERAPEUTICS, US, vol. 77, no. 2, 1 February 2010 (2010-02-01), pages 255-261, XP008127981, ISSN: 0026-895X, DOI: 10.1124/MOL.109.060327 [retrieved on 2009-11-10]

## Description

### Field of the Invention

The present invention relates to 5-substituted-7-[4-(substituted)benzyl]amino-3-isopropyl-pyrazolo[4,3-d]pyrimidines, processes for their preparation, pharmaceutical compositions comprising these compounds and their use for the treatment of antiiflammatory disorders.

### Background Art

Between 145 and 218 millions of Europeans currently suffer from Chronic Inflammatory Diseases, which range from allergic diseases and asthma, to non-allergic autoimmune diseases e.g. inflammatory bowel disease and rheumatoid arthritis and also include chronic fibrotic diseases, including liver cirrhosis. Rheumatoid Arthritis (RA) has been selected as the primary clinical indication in this area. This condition represents a significant disease worldwide, affecting 1 % of the population and this level is likely to increase with changing demographics as it is primarily a disease of older adults (Wolfe AM, Kellgren JH and Masi AT Bull.Rheum.Dis. 19:524-529, 1968). RA is a chronic, systemic, inflammatory disease that primarily affects the joints, but also has several additional manifestations, most notably an increased incidence of cardiovascular diseases. RA is the most common autoimmune inflammatory disease and results in work disability and premature death. Despite 50 years of intense research there has been inadequate progress towards cure and RA therefore still has a significant negative economic impact and is a major burden to Health Services, Citizens and caregivers. Although the aetiology of RA is unknown, like all inflammatory diseases it starts with an acute inflammatory response that later extends into a persistent phase with ensuing tissue damage. Current therapies aim to treat the symptoms of RA and to arrest the disease, but do not provide a cure. Pharmacologic therapy consists of drugs used either singly or in combination and the main drug groupings are: analgesics and non-steroidal anti-inflammatory drugs to relieve pain; disease modifying anti-rheumatic drugs (DMARDS), biologics to target cytokines/immune cells and immunoadsorption to remove autoantibodies.

The biologics target pro-inflammatory cytokines (anti-TNF therapies and IL6 receptor antagonists) and cells of the adaptive immune system (T cells via CTLA4 agonists, B cells via anti-CD20 agonistic antibodies). Importantly, although RA can be held in check by these novel drugs, most notably the anti-TNF therapies, these agents do not represent a cure for RA. In addition, approximately 1 in 3 patients does not respond to anti-TNF therapy (Gartlehner G et al Irheumatol. 33:2398-2408, 2007) and new treatment modalities are therefore urgently required for this significant patient population.

Blocking the contribution of neutrophils and synovial fibroblasts to early RA pathology represents a novel, rational therapeutic option. Neutrophils predominate in the synovial fluid of RA patients and contribute both to the maintenance of inflammation and the tissue damage that leads to pathology in this disease. With the exception of corticosteroids, they are not targeted by current therapies despite the role they play in RA pathology. In addition, the expansion of synovial tissue due to proliferation of fibroblasts also contributes to chronic inflammation and pathology. The expanded tissue eventually invades into cartilage and bone, leading to joint erosion and the pannus also secretes pro-inflammatory cytokines thus maintaining the inflamed state. Again these cells currently receive little attention as therapeutic targets.

An additional driver for the development of new therapies for RA is the current cost of biologics, which are very expensive, thus leading to rationing in many countries, including the EU countries. Treatment of patients in the earlier stages of the disease is also now being actively pursued to delay or avoid the progression of the disease and thus delay or reduce the requirement for biologic therapies. DMARDS applied in early RA can slow progression of the disease in some patients (the 20-25% anti-CCP positive group) and additional therapies and targets are now needed to expand the options for treatment of early RA. WO2007/134828 discloses pyrazolo[4,3-d]pyrimidine derivatives for the treatment of inflammatory disorders.

It is an object of this invention to provide new, very potent, anti-inflammatory compounds, which will be useful to treat RA.

### Disclosure of the Invention

Object of the present invention are 5-substituted-7-[4-(substituted)benzyl]amino-3-isopropylpyrazolo[4,3-d]pyrimidines of the general formula **I** 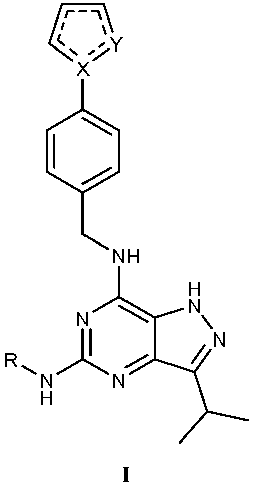 wherein,
**X** is carbon or nitrogen and **Y** is CH, O, S, or N, bonds represented by dashed lines (---) are independently single or double bonds;
**R** is selected from the group consisting of
   - C₂-C₁₀ linear or branched alkyl, optionally substituted by at least one substituent selected from hydroxy and amino substituents, preferably by one hydroxy and/or one amino substituent;
   - (dialkylamino)alkyl group wherein each alkyl is independently selected from C₁-C₁₀ linear or branched alkyl;
   - C₃-C₁₀ cycloalkyl, which is a cyclic or polycyclic alkyl group containing 3 to 10 carbon atoms, optionally substituted by at least one substituent selected from the group hydroxy, C₁-C₄ alkyl, C₁-C₄ alkoxy, C₁-C₄ hydroxyalkyl and amino substituents;
and pharmaceutically acceptable salts thereof, in particular salts with alkali metals, ammonium or amines, or addition salts with acids.

Preferably, the hydroxy-substituted C₂-C₁₀ linear or branched alkyl is selected from the group containing 2-hydroxyethyl, 2(RS, R or S)-hydroxypropyl, 3-hydroxypropyl, 4-hydroxybut-2-(RS, R, or S)-yl, 2-hydroxy-2-methylpropyl, 3-hydroxy-3-methylbutyl, 2,3-dihydroxypropyl, 1-hydroxy-3-methylbut-2-yl, and (3RS)-2-hydroxypent-3-yl.

In another preferred embodiment, the amino-substituted C₂-C₁₀ linear or branched alkyl is selected from the group containing 2-aminoethyl, 3-aminopropyl, 4-aminobutyl, 5-aminopentyl, 6-aminohexyl.

In yet another preferred embodiment, the hydroxy and amino-substituted C₂-C₁₀ linear or branched alkyl is 3-amino-2-hydroxypropyl.

Preferably, the (dialkylamino)alkyl group is selected from the group consisting of (dimethylamino)methyl, 2-(dimethylamino)ethyl, 3-(dimethylamino)propyl, 3-(dimethylamino)butyl, (diethylamino)methyl, 2-(diethylamino)ethyl, 3-(diethylamino)propyl, 4-(diethylamino)butyl.

In another preferred embodiment, the C₃-C₁₀ cycloalkyl is selected from the group containing cyclopropyl, cyclobutyl, cyclopentyl and cyclohexyl.

In one preferred embodiment, the structure 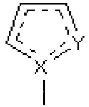 in the position 4 of the benzyl substituent in the general formula **I** contains two double bonds or does not contain any double bond. Preferebly it is selected from the group containing pyrrolidin-1-yl, 1H-pyrazol-1-yl, 1H-pyrrol-2-yl, thiophen-2-yl, furan-2-yl.

When chiral centers are present in the molecule, the present invention encompasses all optically active isomers, their mixtures and racemates. In particular, the compounds of general formula I, having independently at each occurrence (R) or (S) configuration are encompassed by this invention.

The 5-substituted-7-[4-(substituted)benzylamino)-3-isopropylpyrazolo[4,3-d]pyrimidines of the general formula **I** are useful as medicaments. It was found within the framework of the present invention that these compounds combine antiproliferative, antiinflammatory and proapoptotic activities. More specifically, the compounds of general formula I show activity in the treatment of conditions involving inflammation, aberrant cell proliferation and/or apoptosis.

The 5-substituted-7-[4-(substituted)benzylamino)-3-isopropylpyrazolo[4,3-d]pyrimidines of the general formula I are suitable for use in the treatment of inflammatory diseases. In particular, they are useful in the treatment of rheumatoid arthritis.

The 5-substituted-7-[4-(substituted)benzylamino)-3-isopropylpyrazolo[4,3-d]pyrimidines of the general formula **I** are inhibitors of CDK5/7/9 kinases which are the key components involved in regulation of neutrophil and fibroblast division and migration.

The invention also encompasses a pharmaceutical composition, which comprises at least one 5-substituted-7-[4-(substituted)benzylamino)-3-isopropylpyrazolo[4,3-d]pyrimidine of the general formula **I,** and a pharmaceutically acceptable carrier.

Especially preferred are derivatives of general formula **I** bearing substituent R selected from the group comprising: ethyl, propyl, butyl, 2-hydroxyethyl, 3-hydroxypropyl, 2(R)-hydroxypropyl, 2(S)-hydroxypropyl, 4-hydroxybut-2(R)-yl, 4-hydroxybut-2(S)-yl, 4-hydroxybut-2(R,S)-yl, 2-(hydroxy-2-methyl)propyl, 2,3-dihydroxypropyl, 1-hydroxy-3-methylbutyl, (R,S)-2-hydroxypent-3-yl, (R)-2-hydroxypent-3-yl, (S)-2-hydroxypent-3-yl, (*R*)-1-isopropyl-2-hydroxyethyl, (*S*)-1-isopropyl-2-hydroxyethyl, (2-aminoethyl, 3-aminopropyl, 4-aminobutyl, 5-aminopentyl, 6-aminohexyl, 3-amino-2-hydroxypropyl, 1-(dimethylamino)methyl, 2-(dimethylamino)ethyl, 3-(dimethylamino)propyl, 4-(dimethylamino)butyl, 2-(diethylamino)ethyl, 3-(diethylamino)propyl, *cis-*2-aminocyclohexyl, *trans*-2-aminocyclohexyl, *cis,trans*-2-aminocyclohexyl, *cis,trans*-3-aminocyclohexyl, *trans*-4-aminocyclohexyl, *cis*-4-aminocyclohexyl, *cis*,*trans*-4-aminocyclohexyl, *cis*-2-hydroxycyclohexyl, *trans*-2-hydroxycyclohexyl, *cis,trans*-2-hydroxycyclohexyl, *cis,trans*-3-hydroxycyclohexyl, *trans*-4-hydroxycyclohexyl, *cis*-4-hydroxycyclohexyl, *cis,trans*-4-hydroxycyclohexyl.

Preparation of the compounds of the general fomula **I** is starts from 7-chloro-3-isopropyl-5-methylsulfonyl-1*H*-pyrazolo[4,3-d]pyrimidine, in which the chlorine atom is subsituted by 1-[phenyl]methanamine substituted in the position 4 of the phenyl ring, and subsequently the methylsulfonyl group is replaced by R-NH- substituent by reaction with a corresponding amine R-NH₂.

### Pharmaceutical Compositions

Suitable routes for administration include oral, rectal, topical (including dermal, ocular, buccal and sublingual), vaginal and parenteral (including subcutaneous, intramuscular, intravitreous, intravenous, intradermal, intrathecal and epidural). The preferred route of administration will depend upon the condition of the patient, the toxicity of the compound and the site of infection, among other considerations known to the clinician.

The therapeutical composition comprises about 1% to about 95% of the active ingredient, single-dose forms of administration preferably comprising about 20% to about 90% of the active ingredient and administration forms which are not single-dose preferably comprising about 5% to about 20% of the active ingredient. Unit dose forms are, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical compositions of the present invention are prepared in a manner known per se, for example by means of convectional mixing, granulating, coating, dissolving or lyophilising processes.

Preferably, solutions of the active ingredient, and in addition also suspensions or dispersions, especially isotonic aqueous solutions, dispersions or suspensions, are used, it being possible for these to be prepared before use, for example in the case of lyophilised compositions which comprise the active substance by itself or together with a carrier, for example mannitol. The pharmaceutical compositions can be sterilised and/or comprise excipients, for example preservatives, stabilisers, wetting agents and/or emulsifiers, solubilizing agents, salts for regulating the osmotic pressure and/or buffers, and they are prepared in a manner known per se, for example by means of convectional dissolving or lyophilising processes. The solutions or suspensions mentioned can comprise viscosity-increasing substances, preferably selected from sodium carboxymethylcellulose, carboxymethylcellulose, dextran, polyvinylpyrrolidone or gelatin.

Suspensions in oil comprise, as the oily component, the vegetable, synthetic or semi-synthetic oils customary for injection purposes. Oils which may be mentioned are, in particular, liquid fatty acid esters which contain, as the acid component, a long-chain fatty acid having 8 - 22, in particular 12-22, carbon atoms, for example lauric acid, tridecylic acid, myristic acid, pentadecylic acid, palmitic acid, margaric acid, stearic acid, acid, arachidonic acid, behenic acid or corresponding unsaturated acids, for example oleic acid, elaidic acid, euric acid, brasidic acid or linoleic acid, if appropriate with the addition of antioxidants, for example vitamin E, β-carotene or 3,5-di-tert-butyl-4-hydroxytoluene. The alcohol component of these fatty acid esters has not more than 6 carbon atoms and is mono- or polyhydric, for example mono-, di- or trihydric alcohol, for example methanol, ethanol, propanol, butanol, or pentanol, or isomers thereof, but in particular glycol and glycerol. Fatty acid esters are therefore, for example: ethyl oleate, isopropyl myristate, isopropyl palmitate, "Labrafil M 2375" (polyoxyethylene glycerol trioleate from Gattefoseé, Paris), "Labrafil M 1944 CS" (unsaturated polyglycolated glycerides prepared by an alcoholysis of apricot kernel oil and made up of glycerides and polyethylene glycol esters; from Gattefoseé, Paris), "Labrasol" (saturated polyglycolated glycerides prepared by an alcoholysis of TCM and made up of glycerides and polyethylene glycol esters; from Gattefoseé, Paris) and/or "Miglyol 812" (triglyceride of saturated fatty acids of chain length C₈ to C₁₂ from Hüls AG, Germany), and in particular vegetable oils, preferably selected from cottonseed oil, almond oil, olive oil, castor oil, sesame oil, soybean oil and, in particular, groundnut oil.

The preparation of the injection compositions is carried out in the customary manner under sterile conditions, as are bottling, for example in ampoules or vials, and closing of the containers.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients. Suitable carriers are, in particular, fillers, preferably selected from sugars, for example lactose, sucrose, mannitol or sorbitol, cellulose preparations and/or calcium phosphates, for example tricalcium diphosphate, or calcium hydrogen phosphate, and furthermore binders, preferably selected from starches, for example maize, wheat, rice or potato starch, methylcellulose, hydroxypropylmethylcellulose, sodium carboxymethylcellulose and/or polyvinylpyrrolidine, and/or, if desired, desintegrators, preferably selected from the above mentioned starches, and furthermore carboxymethyl-starch, cross-linked polyvinylpyrrolidone, alginic acid or a salt thereof, preferably sodium alginate. Additional excipients are, in particular, flow regulators and lubricants, for example salicylic acid, talc, stearic acid or salts thereof, preferably magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, preferably acetylcellulose phthalate or hydroxypropylmethylcellulose phthalate. Dyes or pigments can be admixed to the tablets or coated tablet coatings, for example for identification or characterisation of different doses of active ingredient.

Pharmaceutical compositions, which can be used orally, are also hard capsules of gelatin and soft, closed capsules of gelatin and a plasticiser, preferably glycerol or sorbitol. The hard capsules can contain the active ingredient in the form of granules, mixed for example with fillers, preferably maize starch, binders and/or lubricants, preferably selected from talc or magnesium stearate, and stabilisers if appropriate. In soft capsules, the active ingredient is preferably dissolved or suspended in suitable liquid excipients, preferably greasy oils, paraffin oil or liquid polyethylene glycols or fatty acid esters of ethylene glycol or propylene glycol, it being likewise possible to add stabilisers and detergents, for example of the polyethylene sorbitan fatty acid ester type.

Other oral forms of administration are, for example, syrups prepared in the customary manner, which comprise the active ingredient, for example, in suspended form and in a concentration of about 5% to 20%, preferably about 10% or in a similar concentration which results in a suitable individual dose, for example, when 5 or 10 ml are measured out. Other forms are, for example, also pulverulent or liquid concentrates for preparing of shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols.

Compositions which are suitable for parental administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and if appropriate stabilisers. The active ingredient can also be present here in the form of a lyophilisate, if appropriate together with excipients, and be dissolved before parenteral administration by addition of suitable solvents. Solutions are used, for example, for parental administration can also be used as infusion solutions. Preferred preservatives are, for example antioxidants, preferably ascorbic acid, or microbicides, preferably sorbic or benzoic acid.

Ointments are oil-in-water emulsions, which comprise not more than 70 %, but preferably 20 - 50 % of water or aqueous phase. The fatty phase consists of, in particular, hydrocarbons, for example vaseline, paraffin oil or hard paraffins, which preferably comprise suitable hydroxy compounds, preferably selected from fatty alcohols or esters thereof, for example cetyl alcohol or wool wax alcohols, preferably wool wax, to improve the water-binding capacity. Emulsifiers are lipophilic substances, preferably selected from sorbitan fatty acid esters (Spans), sorbitan oleate or sorbitan isostearate. Additives to the aqueous phase are, for example, humectants, preferably polyalcohols, for example glycerol, propylene glycol, sorbitol and polyethylene glycol, or preservatives and odoriferous substances.

Fatty ointments are anhydrous and comprise, as the base, in particular, hydrocarbons, for example paraffin, vaseline or paraffin oil, and furthermore naturally occurring or semi-synthetic fats, for example hydrogenated coconut-fatty acid triglycerides, or hydrogenated oils, for example hydrogenated groundnut or castor oil, and furthermore fatty acid partial esters of glycerol, for example glycerol mono- and distearate. They also contain e.g. fatty alcohols, emulsifiers and additives mentioned in connection with ointments which increase the uptake of water.

Creams are oil-in-water emulsions, which comprise more than 50 % of water. Oily bases used are, in particular, fatty alcohols, for example isopropyl myristate, wool wax, beeswax, or hydrocarbons, for example vaseline (petrolatum) or paraffin oil. Emulsifiers are surface-active substances with predominantly hydrophilic properties, preferably corresponding non-ionic emulsifiers, for example fatty acid esters of polyalcohols or ethyleneoxy adducts thereof, preferably polyglyceric fatty acid esters or polyethylene sorbitan fatty acid esters or acidic polyglyceric fatty acid esters (Tween), and furthermore polyoxyethylene fatty alcohol ethers or polyoxyethylene fatty acid esters, or ionic emulsifiers, preferably selected from alkali metal salts of fatty alcohol sulphates, preferably sodium lauryl sulphate, sodium cetyl sulphate or sodium stearyl sulphate, which are usually used in the presence of fatty alcohols, for example cetyl stearyl alcohol or stearyl alcohol. Additives to the aqueous phase are, inter alia, agents which prevent the creams from drying out, for example polyalcohols, preferably selected from glycerol, sorbitol, propylene glycol and polyethylene glycols, and furthermore preservatives and odoriferous substances.

Pastes are creams or ointments containing secretion-absorbing powder constituents, preferably selected from metal oxides, for example titanium oxide or zinc oxide, and in addition talc or aluminium silicates, which have the task of binding the moisture or secretions present.

Foams are administered from pressurised containers and they are liquid oil-in-water emulsions present in aerosol foam. As the propellant gases halogenated hydrocarbons, preferably polyhalogenated alkanes, for example dichlorofluoromethane and dichlorotetrafluoroethane, or, preferably, non-halogenated gaseous hydrocarbons, air, N₂O, or carbon dioxide are used. The oily phases used are, inter alia, those mentioned above for ointments, and the additives mentioned there are likewise used.

Tinctures and solutions usually comprise an aqueous-ethanolic base to which, humectants for reducing evaporation, preferably polyalcohols, for example glycerol, glycols and polyethylene glycol, and re-oiling substances, preferably selected from fatty acid esters with lower polyethylene glycols, i.e. lipophilic substances soluble in the aqueous mixture to substitute the fatty substances removed from the skin with the ethanol, and, if necessary, other excipients and additives are admixed.

This invention further provides veterinary preparations containing at least one active ingredient together with a veterinary carrier. Veterinary carriers are materials for the application of a composition and include solid, liquid or gaseous substances, which are inert or acceptable in veterinary medicine and are compatible with the active ingredient. These veterinary preparations can be administered orally, parenterally or by any other desired way.

The invention also relates to a process or method for treatment of the disease states mentioned above. The compounds can be administered prophylactically or therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition. A daily dose of about 0.1 to about 5 g, preferably 0.5 g to about 2 g, of a compound of the present invention is administered here for a body weight of about 70 kg.

### Brief Description of Drawings

Figure 1 shows effect of compounds **15** and **61** on apoptosis in neutrophils. Isolated human neutrophils were incubated with the compounds for 8 h, subsequently labelled with annexin and then analyzed by flow cytometry.
Figure 2 shows how compound **15** inhibits neutrophil oxygen radical production induced by fMLP (Formyl-Methionyl-Leucyl-Fenylalanin). Isolated human neutrophils were incubated in the presence of prosurvival factor GM-CSF with or without the compound at indicated concentrations for 1 h. A production of ROS during stimulation with fMLP was measured using a luminol-based chemiluminiscent assay.
Figure 3 shows a typical immunodetection of Mcl-1 protein in neutrophils treated by different concentrations of compound 15 (conc. are in nM). Actin was detected as a control to provide comparable application of proteins.

### Examples of Carrying Out the Invention

The following examples serve to illustrate the invention.

Melting points were determined on a Koffler block and are uncorrected. Reagents were from standard commercial sources of analytical grade. Thin layer chromatography (TLC) was carried out using aluminium sheets with silica gel F₂₅₄ from Merck. Spots were visualized under UV light (254 nm). Elementar analaysis (C,H,N) were measure with EA1108 CHN analyser (Thermo Finnigan). ESI or mass spectra were determined using a Waters Micromass ZMD mass spectrometer (cone voltage 20 V). NMR spectra were measured on JEOL 500 ECA instrument at temperature 300 K and frequence 500 MHz (¹H), respectively 125 MHz (¹³C). Column chromatography was performed using Merck silica gel Kieselgel 60 (230 - 400 mesh). All compounds gave satisfactory elemental analyses (± 0.%).

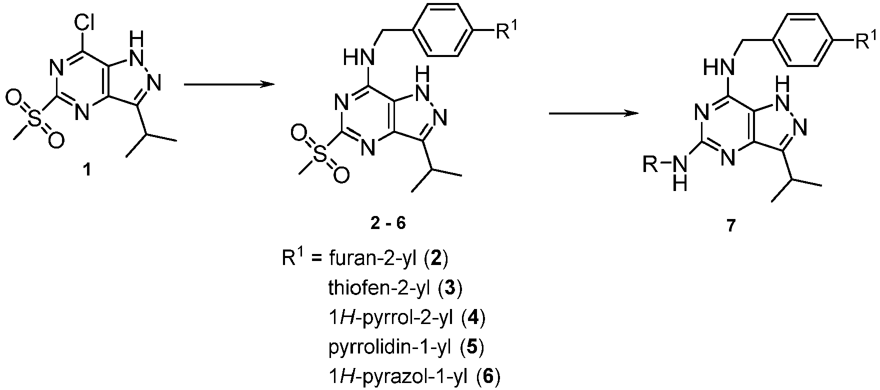

### Example 1

### 3-Isopropyl-5-methylsulfonyl-7-[4-(furan-2-yl)benzyl]amino-1H-pyrazolo[4,3-d]pyrimidine (2)

7-Chloro-3-isopropyl-5-methylsulfonyl-1H-pyrazolo[4,3-d]pyrimidine (1) (17.8 mmol) and ethyldiisopropylamine (22.5 mmol) were heated with stirring in in 80 mL t-BuOH at 60 °C. Solution of 1-[4-(furan-2-yl)phenyl]methanamine (20.9 mmol) in 30 mL t-BuOH (50 °C) was added and the reaction mixture was heated at 60 °C for 1 h. Solvent was removed under vacuum and the residue was purified by column chromatography with gradient of MeOH (1% - 5%) in CHCl3. Chromatography afforded (after evaporation under vacuum) crystals of product in a 65 % yield., mp 180-190°C.

ESI+ m/z 412.1 [M+H]+, ESI- m/z 410.1 [M-H]-.1H (500 MHz; DMSO-d6): 1.32 (d, J = 7.03 Hz, 6H, -CH-(CH3)2); 2.41 (s, 3H, -CH3); 3.33 - 3.35 (m, 1H, -CH-(CH3)2); 4.65 (bs, 2H, -NH-CH2-); 6.58 - 6.61 (m, 1H, ArH); 6.89 - 6.91 (m, 1H, ArH); 7.48 (d, J = 7.03 Hz, 2H, ArH); 7.75 - 7.77 (m, 1H, ArH); 7.92 (d, J = 7.95 Hz, 1H, ArH).

### Example 2

### 3-Isopropyl-5-methylsulfonyl-7-[4-(thiophen-2-yl)benzyl]amino-1H-pyrazolo[4,3-d]pyrimidine (3)

7-Chloro-3-isopropyl-5-methylsulfonyl-1H-pyrazolo[4,3-d]pyrimidine (1) (17.8 mmol) and ethyldiisopropylamine (22.5 mmol) were heated with stirring in in 80 mL t-BuOH at 60 °C. Solution of 1-[4-(thiophen-2-yl)phenyl]methanamine (20.9 mmol) in 30 mL t-BuOH (50 °C) was added and the reaction mixture was heated at 60 °C for 1 h. Solvent was removed under vacuum and the residue was purified by column chromatography with gradient of MeOH (1% - 5%) in CHCl₃. Chromatography afforded (after evaporation under vacuum) crystals of product in a 80 % yield., mp 163-170°C.

ESI+ m/z 428.1 [M+H]⁺, ESI- m/z 426.1 [M-H]⁻.¹H (500 MHz; DMSO-*d*₆): 1.31 (d, *J* = 7.03 Hz, 6H, -CH-(C*H*₃)₂); 2.51 (s, 3H, -CH3); 3.40 - 3.44 (m, 1H, -C*H*-(CH₃)₂); 4.68 (bs, 2H, -NH-C*H*₂-); 7.10 - 7.13 (m, 1H, ArH); 7.38 - 7.40 (m, 1H, ArH); 7.46 (d, *J* = 7.03 Hz, 2H, ArH); 7.70 - 7.72 (m, 1H, ArH); 7.85 (d, *J* = 7.95 Hz, 1H, ArH).

### Example 3

### 3-Isopropyl-5-methylsulfonyl-7-[4-(1H-pyrazol-1-yl)benzyl]amino-1H-pyrazolo[4,3-d]pyrimidine (6)

7-Chloro-3-isopropyl-5-methylsulfonyl-1H-pyrazolo[4,3-d]pyrimidine (1) (17.8 mmol) and ethyldiisopropylamine (22.5 mmol) were heated with stirring in in 80 mL t-BuOH at 60 °C. Solution of 1-[4-(1H-pyrazol-1-yl)phenyl]methanamine (20.9 mmol) in 30 mL t-BuOH (50 °C) was added and the reaction mixture was heated at 60 °C for 1 h. Solvent was removed under vacuum and residue was purified by column chromatography with gradient of MeOH (1% - 5%) in CHCl₃. Chromatography afforded (after evaporation under vacuum) crystals of product in a 80 % yield., after recrystalization from MeOH, mp 195-200°C.

ESI+ m/z 412.2 [M+H]⁺, ESI- m/z 410.2 [M-H]⁻. ¹H (500 MHz; DMSO-*d₆*): 1.39 (d, *J* = 7.03 Hz, 6H, -CH(C*H*₃)₂); 3.26 (s, 3H, -CH₃); 3.37 (sept., *J* = 6.72 Hz, 1H, -C*H*(CH₃)₂); 4.79 (bs, 2H, -CH₂-); 6.51 (dd, *J* = 2.45 Hz, *J* = 1.83 Hz, 1H, ArH); 7.54 (d, *J* = 8.25 Hz, 2H, ArH); 7.70 (d, *J* = 1.53 Hz, 1H, ArH); 7.79 (d, *J* = 8.39 Hz, 2H, ArH); 8.44 (d, *J* = 2.45 Hz, 1H, ArH).

¹³C (125 MHz; DMSO-d6): 21.6; 25.6; 39.5; 43.1; 107.7; 118.2; 118.3; 127.6; 128.2; 128.9; 132.3; 138.8; 140.8; 158.1.

### Example 4

### (R/S)-3-Isopropyl-5-(2-hydroxypropyl)amino-7-[4-(pyrazol-1-yl)benzyl]amino-1H-pyrazolo[4,3-d]pyrimidine (62)

A mixture of 3-isopropyl-5-methylsulfonyl-7-[4-(pyrazol-1-yl)benzyl]amino-1H-pyrazolo[4,3-d]pyrimidine (**6**) (1.42 mmol) and 1-amino-2-propanol (14 mmol) was heated at 135 °C for 8 h in a sealed ampoule. After cooling the reaction mixture was partitioned between H₂O and CHCl₃.

The combined organic phases were dried with sodium sulfate and evaporated under vacuum. The product was purified by column chromatography, stepwise 3%, 5%, 7%, 10% MeOH in CHCl3. Chromatography afforded (after evaporation under vacuum) noncrystalizable amorphous colorless glass foam, yield 33 %.

ESI+ m/z 407.2 [M+H]+, ES- m/z 405.2 [M-H]-. ¹H (500 MHz; DMSO-*d₆*): 1.00 (d, *J* = 6.42 Hz, 3H, -CH-C*H*₃); 1.27 (d, *J* = 6.72 Hz, 6H, -CH-(C*H*₃)₂); 3.07 - 3.13 (m, 2H, -CH₂-); 3.20 - 3.25 (m, 1H, -C*H*-(CH₃)₂); 3.71 - 3.77 (m, 1H, -CH-); 4.66 (bd, *J* = 4.89 Hz, 2H, -NH-C*H*₂-); 6.05 (bs, 1H, -NH-C*H*₂-); 6.47 - 6.48 (m, 1H, ArH); 7.45 (d, *J* = 8.56 Hz, 2H, ArH); 7.66 - 7.68 (m, 1H, ArH); 7.74 - 7.76 (d, *J* = 8.56 Hz, 2H, ArH); 8.40 (d, *J* = 2.45 Hz, 1H, ArH).

¹³C (125 MHz; DMSO-*d₆*): 21.9, 22.1, 26.7, 31.3, 42.9, 49.9, 66.9, 108.0. 108.2, 118.5, 118.9, 128.1, 129.2, 139.2, 141.3

### Example 5

### 3-Isopropyl-5-(2-hydroxy-2-methylpropyl)amino-7-[4-(furan-2-yl)benzyl]amino-1H-pyrazolo[4,3-d]pyrimidine (31)

A mixture of 3-isopropyl-5-methylsulfonyl-7-[4-(furan-2-yl)benzyl]amino-1H-pyrazolo[4,3-d]pyrimidine (**2**) (1.42 mmol) and 1-amino-2-methyl-2-propanol (14 mmol) was heated at 135 °C for 6 h in a sealed ampoule. After cooling the reaction mixture was partitioned between H₂O and CHCl₃. The combined organic phases were dried with sodium sulfate and evaporated under vacuum. The product was purified by column chromatography, stepwise 2%, 4%, 6% MeOH in CHCl₃. Chromatography afforded (after evaporation under vacuum) noncrystalizable amorphous colorless glass foam, yield 23 %.

ESI+ m/z 421.2 [M+H]⁺, ES- m/z 419.2 [M-H]⁻. ¹H (500 MHz; DMSO-*d*₆): 1.06 (s, 6H, -C-(C*H*₃)₂); 1.31 (d, *J* = 7.03 Hz, 6H, -CH-(C*H*₃)₂); 3.24 (sept., *J* = 7.03 Hz, 1H, -C*H*-(CH₃)₂); 3.20 (d, *J* = 5.81 Hz, 2H, -NH-C*H*₂-); 4.68 (d, *J* = 4.89 Hz, 2H, -NH-C*H*₂-); 6.12 (bs, 1H, -N*H*-CH₂-); 6.58 - 6.61 (m, 1H, ArH); 6.89 - 6.91 (m, 1H, ArH); 7.48 (d, *J* = 7.03 Hz, 2H, ArH); 7.75 - 7.77 (m, 1H, ArH); 7.92 (d, *J* = 7.95 Hz, 1H, ArH).

### Example 6

### 3-Isopropyl-5-(2-hydroxy-2-methylpropyl)amino-7-[4-(thiophen-2-yl)benzyl]amino-1H-pyrazolo[4,3-d]pyrimidine (32)

A mixture of 3-isopropyl-5-methylsulfonyl-7-[4-(thiophen-2-yl)benzyl]amino-1H-pyrazolo[4,3-d]pyrimidine (**3**) (1.2 mmol) and 1-amino-2-methyl-2-propanol (12 mmol) was heated at 135 °C for 6 h in a sealed ampoule. After cooling the reaction mixture was partitioned between H₂O and CHCl₃. The combined organic phases were dried with sodium sulfate and evaporated under vacuum. The product was purified by column chromatography, stepwise 2%, 4%, 6% MeOH in CHC1₃. Chromatography afforded (after evaporation under vacuum) noncrystalizable amorphous colorless glass foam, yield 35 %.

ESI+ m/z 437.2 [M+H]⁺, ES- m/z 435.2 [M-H]⁻. ¹H (500 MHz; DMSO-*d*₆): 1.06 (s, 6H, -C-(C*H*₃)₂); 1.31 (d, *J* = 7.03 Hz, 6H, -CH-(C*H*₃)₂); 3.24 (sept., *J* = 7.03 Hz, 1H, -C*H*-(CH₃)₂); 3.20 (d, *J* = 5.81 Hz, 2H, -NH-C*H*₂-); 4.68 (d, *J* = 4.89 Hz, 2H, -NH-C*H*₂-); 6.12 (bs, 1H, -N*H*-CH₂-); 7.11 - 7.13 (m, 1H, ArH); 7.37 - 7.40 (m, 1H, ArH); 7.46 (d, *J* = 7.23 Hz, 2H, ArH); 7.68 - 7.71 (m, 1H, ArH); 7.83 (d, *J* = 8.15 Hz, 1H, ArH).

### Example 7

### 3-Isopropyl-5-(3-amino-2-hydroxypropyl)amino-7-[4-(furan-2-yl)benzyl]amino-1H-pyrazolo[4,3-d]pyrimidine (15)

A mixture of 3-isopropyl-5-methylsulfonyl-7-[4-(furan-2-yl)benzyl]amino-1H-pyrazolo[4,3-d]pyrimidine (**2**) (1.64 mmol), 1,3-diamino-2-propanol (95 mmol) and 1-methyl-2-pyrolidone 2 mL was heated at 135 °C for 6 h in a sealed ampoule. Excess of amine was evaporated at temperature below 70 °C and the residue was partitioned between H₂O and CHCl₃. The combined organic phase was dried with sodium sulfate and were evaporated under vacuum. The product was purified by column chromatography, stepwise 5%, 8%, 11% 14% MeOH in CHCl₃ with a trace of conc. aq. NH₄OH. Chromatography afforded (after evaporation under vacuum) noncrystalizable amorphous colorles solid, yield 24%.

ESI+ m/z 422.2 [M+H]⁺, ESI- m/z 420.2 [M-H]⁻. ¹H (500 MHz; DMSO-*d*₆): 1.25 (d, *J* = 7.03 Hz, 6H, -CH-(C*H*₃)₂); 2.40 - 2.50 (m, 2H, -CH₂-); 3.10 - 3.20 (m, 2H, -CH₂-); 2.28-2.33 (m, 1H, -CH-); 3.45-3.49 (m, 1H, -C*H*-(CH₃)₂); 4.69 (bs, 2H, -NH-C*H*₂-); 6.06 (app. bt, 1H, -N*H*-CH₂-); 6.60 - 6.61 (m, 1H, ArH); 6.87 - 6.91 (m, 1H, ArH); 7.44 (d, *J =* 8.25 Hz, 2H, ArH); 7.75 - 7.77 (m, 1H, ArH); 8.01 (d, *J* = 8.56 Hz, 2H, ArH).

### Example 8

### 3-Isopropyl-5-(3-amino-2-hydroxypropyl)amino-7-[4-(1H-pyrazol-1-yl)benzyl]amino-1H-pyrazolo[4,3-d]pyrimidine (61)

A mixture of 3-isopropyl-5-methylsulfonyl-7-[4-(1H-pyrazol-1-yl)benzyl]amino-1H-pyrazolo[4,3-d]pyrimidine (**6**) (1.64 mmol), 1,3-diamino-2-propanol (95 mmol) and 1-methyl-2-pyrolidone 2 mL was heated at 135 °C for 6 h in a sealed ampoule. Excess of amine was evaporated at temperature below 70 °C and the residue was partitioned between H₂O and CHCl. The combined organic phase was dried with sodium sulfate and were evaporated under vacuum. The product was purified by column chromatography, stepwise 5%, 8%, 11% 14% MeOH in CHCl₃ with a trace of conc. aq. NH₄OH. Chromatography afforded (after evaporation under vacuum) noncrystalizable amorphous colorles solid, yield 35%.

ESI+ m/z 422.2 [M+H]⁺, ESI- m/z 420.2 [M-H]⁻. ¹H (500 MHz; DMSO-*d*₆): 1.25 (d, *J* = 7.03 Hz, 6H, -CH-(C*H*₃)₂); 2.40 - 2.50 (m, 2H, -CH₂-); 3.10 - 3.20 (m, 2H, -CH₂-); 2.28-2.33 (m, 1H, -CH-); 3.45-3.49 (m, 1H, -C*H*-(CH₃)₂); 4.69 (bs, 2H, -NH-C*H*₂-); 6.06 (app. bt, 1H, -N*H*-CH₂-); 6.49 (dd, *J* = 2.45 Hz, *J* = 1.83 Hz, 1H, ArH); 7.40 (d, *J* = 8.56 Hz, 2H, ArH); 7.61 (d, *J* = 1.53 Hz, 1H, ArH); 7.80 (d, *J* = 8.56 Hz, 1H, ArH); 8.39 (d, *J* = 2.14 Hz, 1H, ArH).

### Example 9

### 3-Isopropyl-5-(trans-2-aminocyclohexyl)amino-7-[4-(furan-2-yl)benzyl]amino-1H-pyrazolo[4,3-d]pyrimidine (17)

Solution of 3-isopropyl-5-methylsulfonyl-7-[4-(furan-2-yl)benzyl] amino-1H-pyrazolo[4,3-d]pyrimidine (**2**) (3.32 mmol) in 1,2-trans-diaminocyclohexane (68 mmol) in sealed ampoule was heated at 145 °C for 20 h. Excess of amine was evaporated at temperature below 70 °C and the residue was partitioned between H₂O and CHCl₃. The combined organic phase was dried with sodium sulfate and were evaporated under vacuum. The product was purified by column chromatography, stepwise 4%, 8%, 11%, 14% MeOH in CHCl₃ with a trace of conc. aq. NH₄OH. Chromatography afforded (after evaporation under vacuum) noncrystalizable amorphous colorless glass foam, yield 13%.

ESI+ m/z 446.2 [M+H]⁺, ESI- m/z 444.3 [M-H]⁻.¹H (500 MHz; CDCl₃): 1.13 - 1.20 (m, 4H, -CH₂-); 1.25 (bs, 6H, -CH-(C*H*₃)₂); 1.57 - 1.59 (m, 2H, -CH₂-); 1.86 (bs, 2H, -CH₂-); 2.47 (bs, 1H, -CH-); 3.15 (sept., *J* = 6.42 Hz, 1H, -C*H*-(CH₃)₂); 3.56 (bs, 1H, -CH-); 4.57 (bs, 2H, -NH-C*H*₂-); 6.58 - 6.61 (m, 1H, ArH); 6.89 - 6.91 (m, 1H, ArH); 7.48 (d, *J =* 7.13 Hz, 2H, ArH); 7.75 - 7.77 (m, 1H, ArH); 7.92 (d, *J* = 7.15 Hz, 1H, ArH).

### Example 10

*3-Isopropyl-5-(trans-4-aminocyclohexyl)amino-7-[4-(furan-2-yl)benzyl]amino-1H-pyrazolo[4,3-d]pyrimidine (20)*

3-Isopropyl-5-methylsulfonyl-7-[4-(furan-2-yl)benzyl]amino-1H-pyrazolo[4,3-d]pyrimidine (**2**) (0.71 mmol) in melted 1,4-trans-diaminocyclohexane (44 mmol) in sealed ampoule was heated at 145 °C for 20 h. Excess of amine was evaporated at temperature below 70 °C and the residue was partitioned between H₂O and CHCl₃. The combined organic phase was dried with sodium sulfate and were evaporated under vacuum. The product was purified by column chromatography, stepwise 4%, 8%, 11%, 14% MeOH in CHCl₃ with a trace of conc. aq. NH₄OH. Chromatography afforded (after evaporation under vacuum) noncrystalizable amorphous colorless glass solid, yield 15%.

ESI+ m/z 446.2 [M+H]⁺, ESI- m/z 444.3 [M-H]⁻. ¹H (500 MHz; DMSO-*d*₆): 1.08 - 1.18 (m, 4H, 2x -CH₂-); 1.27 (d, *J* = 7.03 Hz, 6H, -CH-(C*H*₃)₂); 1.71 - 1.72 (m, 2H, -CH₂-); 1.85 - 1.87 (m, 2H, -CH₂-); 2.50 - 2.52 (m, 1H, -C*H*-NH-); 3.11 (sept., *J* = 7.03 Hz, 1H, -C*H*-(CH₃)₂); 3.54 - 3.56 (m, 1H, -C*H*-NH-); 4.68 (bd, *J* = 4.58 Hz, 2H, -NH-C*H*₂-); 5.69 (bd, *J* = 7.34 Hz, 1H, -N*H*-CH-); 6.58 - 6.61 (m, 1H, ArH); 6.89 - 6.91 (m, 1H, ArH); 7.48 (d, *J* = 7.13 Hz, 2H, ArH); 7.75 - 7.77 (m, 1H, ArH); 7.92 (d, *J* = 7.15 Hz, 1H, ArH).

**Table 1: Compounds Prepared by the Method of Examples 4-10.**

| | | | | | |
|---|---|---|---|---|---|
| 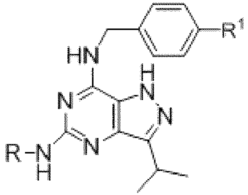 | | | | | |

| No | R | R¹ | CHN ANALYSES [%] | MS (ZMD)-ANALYSES | |
|---|---|---|---|---|---|
| | | | | [M-H]⁻ a) | [M+H]⁺ b) |
| **7** | 2-hydroxyethyl | furan-2-yl | C=64.26; H=6.14; N=21.40 | 391.2 | 393.2 |
| **8** | (R/S)-2-hydroxypropyl | furan-2-yl | C=65.05; H=6.40; N=20.70 | 405.2 | 407.2 |
| **9** | (R)-2-hydroxypropyl | furan-2-yl | C=65.00; H=6.45; N=20.65 | 405.2 | 407.2 |
| **10** | (S)-2-hydroxypropyl | furan-2-yl | C=64.92; H=6.48; N=20.72 | 405.2 | 407.2 |
| **11** | 3-hydroxypropyl | furan-2-yl | C=65.02; H=6.48; N=20.68 | 405.2 | 407.2 |
| **12** | (R/S)-4-hydroxybut-2-yl | furan-2-yl | C=65.72; H=6.71; N=19.99 | 419.3 | 421.2 |
| **13** | (R)-4-hydroxybut-2-yl | furan-2-yl | C=65.70; H=6.75; N=19.97 | 419.3 | 421.2 |
| **14** | (S) -4-hydroxybut-2-yl | furan-2-yl | C=65.68; H=6.69; N=20.02 | 419.3 | 421.2 |
| **15** | 3-amino-2-hydroxypropyl | furan-2-yl | C=62.69; H=6.46; N=23.30 | 420.2 | 422.2 |
| **16** | *cis*-2-aminocyclohexyl | furan-2-yl | C=67.45; H=7.00; N=22.01 | 444.2 | 446.2 |
| **17** | *trans*-2-aminocyclohexyl | furan-2-yl | C=67.39; H=6.99; N=22.02 | 444.3 | 446.2 |
| **18** | *cis,trans*-2-aminocyclohexyl | furan-2-yl | C=67.45; H=7.07; N=21.98 | 444.3 | 446.2 |
| **19** | *cis,trans*-3-aminocyclohexyl | furan-2-yl | C=67.37; H=7.01; N=22.04 | 444.2 | 446.2 |
| **20** | *trans*-4-aminocyclohexyl | furan-2-yl | C=67.40; H=7.01; N=22.03 | 444.3 | 446.2 |
| **21** | *trans*-2-hydroxycyclohexyl | furan-2-yl | C=67.29; H=6.78; N=18.79 | 445.2 | 447.2 |
| **22** | *trans-*3-hydroxycyclohexyl | furan-2-yl | C=67.24; H=6.72; N=18.89 | 445.2 | 447.2 |
| **23** | *trans*-4-hydroxycyclohexyl | furan-2-yl | C=67.21; H=6.75; N=18.84 | 445.2 | 447.2 |
| **24** | (R)-1-(hydroxymethyl)propyl | furan-2-yl | C=65.70; H=6.73; N=20.01 | 419.2 | 421.2 |
| **25** | (S)-1-(hydroxymethyl)propyl | furan-2-yl | C=65.68 H=6.68; N=19.96 | 419.2 | 421.2 |
| **26** | 2-aminoethyl | furan-2-yl | C=64.45; H=6.46; N=25.06 | 390.2 | 392.2 |
| **27** | 3-aminopropyl | furan-2-yl | C=65.18; H=6.73; N=24.20 | 404.2 | 406.2 |
| **28** | 4-aminobutyl | furan-2-yl | C=65.82; H=7.00; N=23.38 | 418.2 | 420.2 |
| **29** | 5-aminopentyl | furan-2-yl | C=66.47; H=7.22; N=22.62 | 432.3 | 434.3 |
| **30** | 6-aminohexyl | furan-2-yl | C=67.11; H=7.45; N=21.95 | 446.3 | 448.3 |
| **31** | 2-hydroxy-2-methylpropyl | furan-2-yl | C=65.69; H=6.70; N=19.96 | 419.2 | 421.2 |
| **32** | 2-hydroxy-2-methylpropyl | thiophen-2-yl | C=63.25; H=6.49; N=19.30 | 435.2 | 437.2 |
| **33** | 3-hydroxy-3-methylbutyl | thiophen-2-yl | C=63.99; H=6.75; N=18.66 | 449.3 | 451.2 |
| **34** | 2,3-dihydroxypropyl | thiophen-2-yl | C=60.21; H=6.05; N=19.20 | 437.2 | 439.2 |
| **35** | 1-hydroxy-3-methylbut-2-yl | thiophen-2-yl | C=63.97; H=6.73; N=18.59 | 449.2 | 451.2 |
| **36** | (R/S)-2-hydroxypent-3-yl | thiophen-2-yl | C=63.95; H=6.80; N=18.70 | 449.2 | 451.2 |
| **37** | (R)-2-hydroxypent-3-yl | thiophen-2-yl | C=63.92; H=6.71; N=18.55 | 449.2 | 451.2 |
| **38** | (S)-2-hydroxypent-3-yl | thiophen-2-yl | C=63.95; H=6.75; N=18.65 | 449.2 | 451.2 |
| **39** | 2-hydroxyethyl | thiophen-2-yl | C=61.75; H=5.94; N=20.59 | 407.2 | 409.2 |
| **40** | (R/S)-2-hydroxypropyl | thiophen-2-yl | C=62.54; H=6.24; N=19.90 | 421.2 | 423.2 |
| **41** | (R)-2-hydroxypropyl | thiophen-2-yl | C=62.50; H=6.19; N=19.92 | 421.2 | 423.2 |
| **42** | (S)-2-hydroxypropyl | thiophen-2-yl | C=62.53; H=6.21; N=19.88 | 421.2 | 423.2 |
| **43** | 3-hydroxypropyl | thiophen-2-yl | C=62.52; H=6.18; N=19.92 | 421.2 | 423.2 |
| **44** | (R/S)-4-hydroxybut-2-yl | thiophen-2-yl | C=63.30; H=6.47; N=19.25 | 435.2 | 437.2 |
| **45** | (R)-4-hydroxybut-2-yl | thiophen-2-yl | C=63.27; H=6.49; N=19.26 | 435.2 | 437.2 |
| **46** | (S)-4-hydroxybut-2-yl | thiophen-2-yl | C=63.28; H=6.46; N=19.22 | 435.2 | 437.2 |
| **47** | 2-aminoethyl | 1H-pyrrol-2-yl | C=64.59; H=6.78; N=28.64 | 389.3 | 391.3 |
| **48** | 3-aminopropyl | 1H-pyrrol-2-yl | C=65.33; H=6.99; N=27.72 | 403.2 | 405.2 |
| **49** | 4-aminobutyl | 1H-pyrrol-2-yl | C=66.08; H=7.25; N=26.78 | 417.3 | 419.3 |
| **50** | 5-aminopentyl | 1H-pyrrol-2-yl | C=66.69; H=7.45; N=25.92 | 431.3 | 433.3 |
| **51** | 6-aminohexyl | 1H-pyrrol-2-yl | C=67.26; H=7.69; N=25.10 | 445.3 | 447.3 |
| **52** | 1-(dimethylamino)methyl | 1H-pyrazol-1-yl | C=62.25; H=6.72; N=31.11 | 404.2 | 406.2 |
| **53** | 2-(dimethylamino)ethyl | 1H-pyrazol-1-yl | C=62.96; H=6.99; N=30.05 | 418.3 | 420.3 |
| **54** | 3-(dimethylamino)propyl | 1H-pyrazol-1-yl | C=63.70; H=7.25; N=29.08 | 432.3 | 434.3 |
| **55** | 4-(dimethylamino)butyl | 1H-pyrazol-1-yl | C=64.42; H=7.45; N=28.17 | 446.3 | 448.3 |
| **56** | 2-aminoethyl | 1H-pyrazol-1-yl | C=61.39; H=6.48; N=32.24 | 390.2 | 392.2 |
| **57** | 3-aminopropyl | 1H-pyrazol-1-yl | C=62.23; H=6.70; N=31.07 | 404.2 | 406.2 |
| **58** | 4-aminobutyl | 1H-pyrazol-1-yl | C=62.98; H=6.99; N=30.08 | 418.3 | 420.3 |
| **59** | 5-aminopentyl | 1H-pyrazol-1-yl | C=63.72; H=7.22; N=29.10 | 432.3 | 434.3 |
| **60** | 6-aminohexyl | 1H-pyrazol-1-yl | C=64.46; H=7.47; N=28.17 | 446.3 | 448.3 |
| **61** | 3 -amino-2-hydroxypropyl | 1H-pyrazol-1-yl | C=59.85; H=6.45; N=29.95 | 420.2 | 422.2 |
| **62** | (R/S)-2-hydroxypropyl | 1H-pyrazol-1-yl | C=62.15; H=6.45; N=27.55 | 405.2 | 407.2 |
| **63** | 2-(diethylamino)ethyl | pyrrolidin-1-yl | C=66.61; H=8.49; N=24.82 | 449.3 | 451.3 |
| **64** | 3 -(diethylamino)propyl | pyrrolidin-1-yl | C=67.20; H=8.70; N=24.16 | 463.3 | 465.3 |
| **65** | 3-(diethylamino)butyl | pyrrolidin-1-yl | C=67.73; H=8.86; N=23.40 | 477.4 | 479.3 |
| **66** | 2-hydroxy-2-methylpropyl | pyrrolidin-1-yl | C=65.25; H=7.88; N=23.18 | 422.3 | 424.3 |
| **67** | 3-hydroxy-3-methylbutyl | pyrrolidin-1-yl | C=65.90; H=8.05; N=22.46 | 436.3 | 438.3 |
| **68** | 2,3-dihydroxypropyl | pyrrolidin-1-yl | C=62.13; H=7.35; N=23.02 | 424.2 | 426.2 |
| **69** | 1-hydroxy-3-methylbut-2-yl | pyrrolidin-1-yl | C=65.90; H=8.09; N=22.45 | 436.3 | 438.3 |

| | | | | | |
|---|---|---|---|---|---|
| a) solution: MeOH p.a. + HCOOH b) solution: MeOH p.a. + H₂O + NH₃ | | | | | |

### Example 11

### Measurement of neutrophil apoptosis

The neutrophils were isolated from the peripheral blood of healthy human donors as described previously (Afford et al 1992). Briefly, blood was collected into tubes containing EDTA followed by usage of 2% dextran solution for sedimentation of erythrocytes. The neutrophils were then isolated from leukocyte layer using a discontinuous Percoll density gradient centrifugation (Sigma- Aldrich, Poole, UK) and resuspended in RPMI 1640 medium (Sigma- Aldrich) supplemented with 10% heat-inactivated fetal bovine serum (Sera Laboratories International, Bolney, UK), 2 mM glutamine, 100 U/ml penicillin and 100 µg/ml streptomycin (Sigma-Aldrich). The purity of isolated neutrophils was assessed by Giemsa staining (Diff-Quick, Baxter Healthcare, UK) and light microscopy and was routinely greater than 97%. The neutrophils were then cultured in the presence of novel compounds (single concentration of 10 nM, or several increasing concentrations ranging from 10 nM to 1 µM) and other stimulating factors for 8 h.

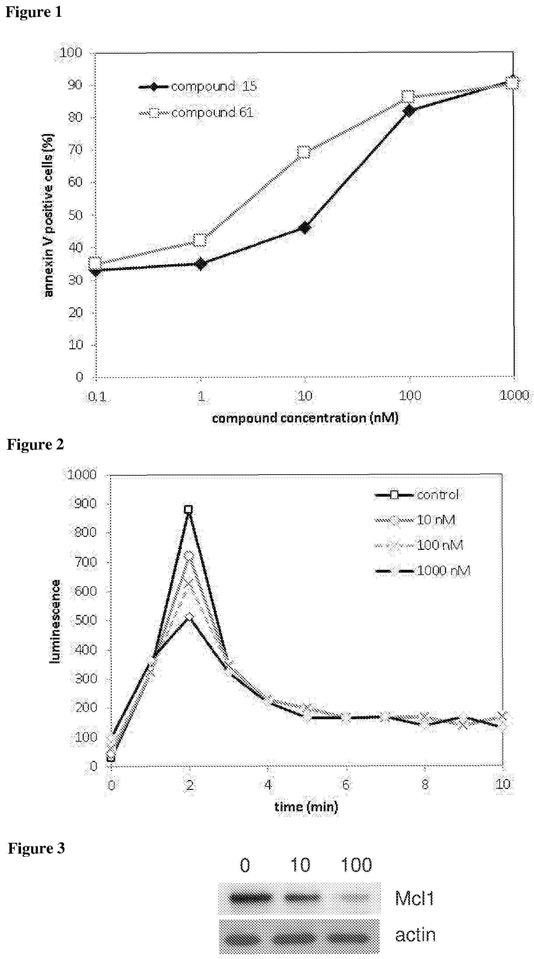

For evaluation of early apoptosis, neutrophils were double stained with anti-Annexin V FITC-conjugated antibody (BD Pharmingen) binding extracellular phosphatidylserine and propidium iodide detecting late apoptotic and necrotic cells. After appropriate treatment, cells were washed with PBS and then resuspended in 100 µl of Annexin V buffer (10 mM HEPES, pH 7.4; 140 mM NaCl; 2.5 mM CaCl₂). 1 µl of anti-Annexin V FITC-conjugated antibody was added and cells were then incubated at room temperature for 10 minutes followed by addition of propidium iodide at final concentration of 5 µg/ml. Fluorescence was analyzed by flow cytometry (BD Accuri C6 Flow Cytometer, Accuri Cytometers Inc.) with Annexin V positive / PI negative cells taken as apoptotic. Resulting data are summarized in Table 2. Some novel compounds were also assayed at several doses and the Figure 1 shows clear dose-dependent effect.

Proapoptotic action of active compounds was confirmed by morphological analysis of neutrophil nuclei using Giemsa staining. After treatment with novel compounds most of the cells appeared to be apoptotic. The effect was clearly dose-dependent.

**Table 2. Induction of apoptosis in neutrophils by novel compounds (10 nM).**

| **compound** | **% of apoptotic cells** |
|---|---|
| 1 | 32 |
| 2 | 41 |
| 3 | 58 |
| 4 | 46 |
| 5 | 40 |
| 6 | 36 |
| 7 | 47 |
| 15 | 52 |
| 30 | 39 |
| 31 | 50 |
| 32 | 48 |
| 33 | 36 |
| 34 | 52 |
| 42 | 57 |
| 44 | 38 |
| 45 | 33 |
| 58 | 44 |
| 59 | 49 |
| 60 | 60 |
| 61 | 40 |
| 63 | 39 |
| 65 | 56 |
| 66 | 34 |

### Example 18

### Superoxide generation assay

Levels of reactive oxygen species (ROS) generated by neutrophils were measured using a luminol-based chemiluminescent assay (Gyllenhammar 1987). After treatment, neutrophils were resuspended in Hank's Balanced Salt Solution (HBSS) containing calcium and magnesium (Gibco) and transferred to opaque 96 well plates (Greiner Bio-One GmbH). Luminol was then added to each well at final concentration of 100 µM followed by a measuring of chemiluminescence in response to the stimulus fMLP (2.5 µM) at 1 min intervals for 30 min by means of a luminometer (Centro LB 960, Berthold Technologies, Bad Wildbad, Germany). The effect of compounds was clearly dose-dependent, as shown in Figure 2.

### Example 19

### Downregulation of Mcl-1 protein

Neutrophils were after treatment resuspended in SDS-PAGE sample buffer (125 mM HCl, pH 6.8; 5% glycerol; 2% SDS; 1% β-mercaptoethanol; 0.003% bromophenol blue), prepared lysates were incubated for 10 min at 100 °C, then separated by means of SDS-PAGE and blotted onto PVDF membranes. Non-specific protein binding was blocked using 5% BSA. The membranes were consequently incubated with primary antibodies against Mcl-1 (1:500), Bak (1:500) and β-actin (1:1000) overnight at 4 °C followed by incubation with appropriate secondary antibodies (ECL anti-rabbit or anti-mouse IgG; GE Healthcare, Sweden) for 1 hour at room temperature. The proteins were visualized by ECL according to manufacturer's instructions (GeneFlow, UK). Densitometric analyses were performed using Multi Gauge software (FujiFilm).

After 8 h treatment with novel compounds the neutrophils showed gradual decrease in Mcl-1 protein levels (Figure 3). A proapoptotic protein Bak showed concentration-dependent growth.

### Example 20

### Dry Capsules

5000 capsules, each of which contains 0.25 g of one of the compounds of the formula I mentioned in the preceding or following Examples as active ingredient, are prepared as follows:

**Composition**

| | |
|---|---|
| Active ingredient | 1250 g |
| Talc | 180 g |
| Wheat starch | 120 g |
| Magnesium stearate | 80 g |
| Lactose | 20 g |

Preparation process: The powdered substances mentioned are pressed through a sieve of mesh width 0.6 mm. Portions of 0.33 g of the mixture are transferred to gelatine capsules with the aid of a capsule-filling machine.

### Example 21

### Soft Capsules

5000 soft gelatine capsules, each of which contains 0.05 g of one of the compounds of the formula **I** mentioned in the preceding or following Examples as active ingredient, are prepared as follows:

**Composition**

| | |
|---|---|
| Active ingredient | 250 g |
| Lauroglycol | 2 litres |

Preparation process: The powdered active ingredient is suspended in Lauroglykol® (propylene glycol laurate, Gattefossé S.A., Saint Priest, France) and ground in a wet-pulveriser to a particle size of about 1 to 3 µm. Portions of in each case 0.419 g of the mixture are then transferred to soft gelatine capsules by means of a capsule-filling machine.

### Example 22

### Soft Capsules

5000 soft gelatine capsules, each of which contains 0.05 g of one of the compounds of the formula **I** mentioned in the preceding or following Examples as active ingredient, are prepared as follows:

**Composition**

| | |
|---|---|
| Active ingredient | 250 g |
| PEG 400 | 1 litre |
| Tween 80 | 1 litre |

Preparation process: The powdered active ingredient is suspended in PEG 400 (polyethylene glycol of Mr between 380 and about 420, Sigma, Fluka, Aldrich, USA) and Tween® 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 mm. Portions of in each case 0.43 g of the mixture are then transferred to soft gelatine capsules by means of a capsule-filling machine.

## Claims

1. 5-Substituted-7-[4-(substituted)benzyl]amino-3-isopropylpyrazolo[4,3-d]pyrimidines of general formula **I** 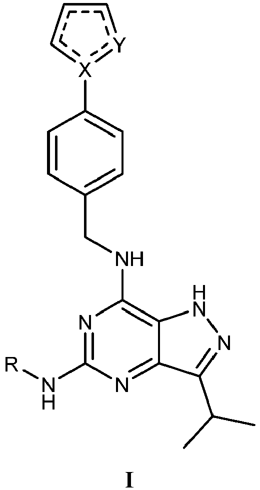 wherein,
**X** is carbon or nitrogen and **Y** is CH, O, S, or N, bonds represented by dashed lines (---) are independently single or double bonds;
**R** is selected from the group consisting of
- C₂-C₁₀ linear or branched alkyl, optionally substituted by at least one substituent selected from hydroxy and amino substituents, preferably by one hydroxy and/or one amino substituent;
- (dialkylamino)alkyl group wherein each alkyl is independently selected from C₁-C₁₀ linear or branched alkyl;
- C₃-C₁₀ cycloalkyl, which is a cyclic or polycyclic alkyl group containing 3 to 10 carbon atoms, optionally substituted by at least one substituent selected from the group hydroxy, C₁-C₄ alkyl, C₁-C₄ alkoxy, C₁-C₄ hydroxyalkyl and amino substituents;
and pharmaceutically acceptable salts thereof, in particular salts with alkali metals, ammonium or amines, or addition salts with acids.

2. 5-Substituted-7-[4-(substituted)benzyl]amino-3-isopropylpyrazolo[4,3-d]pyrimidines of general formula **I** according to claim 1, wherein **R** is selected from the group comprising ethyl, propyl, butyl, 2-hydroxyethyl, 3-hydroxypropyl, 2(R)-hydroxypropyl, 2(S)-hydroxypropyl, 4-hydroxybut-2(R)-yl, 4-hydroxybut-2(S)-yl, 4-hydroxybut-2(R,S)-yl, 2-(hydroxy-2-methyl)propyl, 2,3-dihydroxypropyl, 1-hydroxy-3-methylbutyl, (R,S)-2-hydroxypent-3-yl, (R)-2-hydroxypent-3-yl, (S)-2-hydroxypent-3-yl, (*R*)-1-isopropyl-2-hydroxyethyl, (*S*)-1-isopropyl-2-hydroxyethyl, 2-aminoethyl, 3-aminopropyl, 4-aminobutyl, 5-aminopentyl, 6-aminohexyl, 3-amino-2-hydroxypropyl, 1-(dimethylamino)methyl, 2-(dimethylamino)ethyl, 3-(dimethylamino)propyl, 4-(dimethylamino)butyl, 2-(diethylamino)ethyl, 3-(diethylamino)propyl, *cis*-2-aminocyclohexyl, *trans*-2-aminocyclohexyl, *cis,trans*-2-aminocyclohexyl, *cis*,*trans*-3-aminocyclohexyl, *trans*-4-aminocyclohexyl, *cis*-4-aminocyclohexyl, *cis,trans*-4-aminocyclohexyl, *cis*-2-hydroxycyclohexyl, *trans*-2-hydroxycyclohexyl, *cis,trans*-2-hydroxycyclohexyl, *cis,trans*-3-hydroxycyclohexyl, *trans-*4-hydroxycyclohexyl, *cis*-4-hydroxycyclohexyl, *cis,trans*-4-hydroxycyclohexyl.

3. 5-Substituted-7-[4-(substituted)benzyl]amino-3-isopropylpyrazolo[4,3-d]pyrimidines of the general formula **I** according to any one of claims 1 or 2, wherein the structure 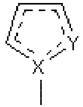 is selected from the group containing pyrrolidin-1-yl, 1H-pyrazol-1-yl, 1H-pyrrol-2-yl, thiophen-2-yl, furan-2-yl.

4. 5-Substituted-7-[4-(substituted)benzyl]amino-3-isopropylpyrazolo[4,3-d]pyrimidines of the general formula **I** according to any one of the preceding claims for use as medicaments.

5. 5-Substituted-7-[4-(substituted)benzyl]amino-3-isopropylpyrazolo[4,3-d]pyrimidines of the general formula **I** according to any one of claims 1 to 3 for use in the treatment of inflammatory diseases.

6. 5-Substituted-7-[4-(substituted)benzyl]amino-3-isopropylpyrazolo[4,3-d]pyrimidines of the general formula **I** according to any one of claims 1 to 3 for use in in the treatment of rheumatoid arthritis.

7. A pharmaceutical composition, **characterized in that** it comprises at least one 5-substituted-7-[4-(substituted)benzyl]amino-3-isopropylpyrazolo[4,3-d]pyrimidine of the general formula **I** according to any one of claims 1 to 3, and at least one pharmaceutically acceptable carrier.

## Patentansprüche

1. 5-Substituierte-7-[4-(substituierte)benzyl]amino-3-isopropylpyrazolo[4,3-d]pyrimidine der allgemeinen Formel **I** 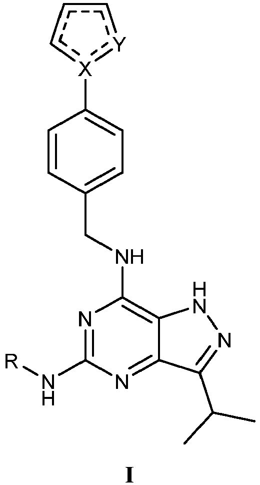 worin,
**X** ist Kohlenstoff oder Stickstoff und **Y** ist CH, O, S oder N, durch gestrichelte Linien (---) dargestellte Bindungen sind unabhängig voneinander Einfach- oder Doppelbindungen;
**R** ist ausgewählt aus der Gruppe bestehend aus
- lineares oder verzweigtes C₂-C₁₀-Alkyl, gegebenenfalls substituiert durch mindestens einen Substituenten, ausgewählt aus Hydroxy- und Aminosubstituenten, vorzugsweise durch einen Hydroxy- und/oder einen Aminosubstituenten;
- (Dialkylamino)alkylgruppe, wobei jedes Alkyl unabhängig ausgewählt ist aus linearem oder verzweigtem C₁-C₁₀-Alkyl;
- C₃-C₁₀-Cycloalkyl, welches ist eine cyclische oder polycyclische Alkylgruppe mit 3 bis 10 Kohlenstoffatomen, gegebenenfalls substituiert durch mindestens einen Substituenten, ausgewählt aus der Gruppe unfassend Hydroxy, C₁-C₄-Alkyl, C₁-C₄-Alkoxy, C₁-C₄-Hydroxyalkyl und Amino Substituenten;
und pharmazeutisch verträgliche Salze davon, insbesondere Salze mit Alkalimetallen, Ammonium oder Aminen oder Additionssalze mit Säuren.

2. 5-Substituierte-7-[4-(substituierte)benzyl]amino-3-isopropylpyrazolo[4,3-d]pyrimidine der allgemeinen Formel I nach Anspruch 1, wobei **R** ausgewählt ist aus der Gruppe umfassend Ethyl, Propyl, Butyl, 2-Hydroxyethyl, 3-Hydroxypropyl, 2(R)-Hydroxypropyl, 2(S)-Hydroxypropyl, 4-Hydroxybut-2(R)-yl, 4-Hydroxybut-2(S)-yl, 4-Hydroxybut-2(R,S)-yl, 2-(Hydroxy-2-methyl)propyl, 2,3-Dihydroxypropyl, 1-Hydroxy-3-methylbutyl, (R,S)-2-Hydroxypent-3-yl, (R)-2-Hydroxypent-3-yl, (S)-2-Hydroxypent-3-yl, (R)-1-Isopropyl-2-hydroxyethyl, (S)-1-Isopropyl-2-hydroxyethyl, 2-Aminoethyl, 3-Aminopropyl, 4-Aminobutyl, 5-Aminopentyl, 6-Aminohexyl, 3-Amino-2-hydroxypropyl, 1-(Dimethylamino)methyl, 2-(Dimethylamino)ethyl, 3-(Dimethylamino)propyl, 4-(Dimethylamino)butyl, 2-(Diethylamino)ethyl, 3-(Diethylamino)propyl, *cis*-2-Aminocyclohexyl, *trans-*2-Aminocyclohexyl, *cis,trans-*2-Aminocyclohexyl, *cis,trans*-3-Aminocyclohexyl, *trans*-4-Aminocyclohexyl, *cis*-4-Aminocyclohexyl, *cis*,*trans*-4-Aminocyclohexyl, *cis*-2-Hydroxycyclohexyl, *trans*-2-Hydroxycyclohexyl, *cis,trans*-2-Hydroxycyclohexyl, *cis,trans*-3-Hydroxycyclohcxyl, *trans*-4-Hydroxycyclohexyl, *cis*-4-Hydroxycyclohexyl, *cis,trans*-4-Hydroxycyclohexyl.

3. 5-Substituierte-7-[4-(substituierte)benzyl]amino-3-isopropylpyrazolo[4,3-d]pyrimidine der allgemeinen Formel I nach einem der Ansprüche 1 oder 2, wobei die Struktur 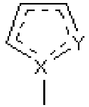 ausgewählt ist aus die Gruppe umfassend Pyrrolidin-1-yl, 1H-Pyrazol-1-yl, 1H-Pyrrol-2-yl, Thiophen-2-yl, Furan-2-yl.

4. 5-Substituierte-7-[4-(substituierte)benzyl]amino-3-isopropylpyrazolo[4,3-d]pyrimidine der allgemeinen Formel I gemäß einem der vorhergehenden Ansprüche zur Verwendung als Arzneimittel.

5. 5-Substituierte-7-[4-(substituierte)benzyl]amino-3-isopropylpyrazolo[4,3-d]pyrimidine der allgemeinen Formel I nach einem der Ansprüche 1 bis 3 zur Verwendung bei der Behandlung von Entzündunglichen Krankheiten.

6. 5-Substituierte-7-[4-(substituierte)benzyl]amino-3-isopropylpyrazolo[4,3-d]pyrimidine der allgemeinen Formel I nach einem der Ansprüche 1 bis 3 zur Verwendung bei der Behandlung von rheumatoide Arthritis.

7. Pharmazeutische Zusammensetzung, die **dadurch gekennzeichnet ist, dass** sie mindestens ein 5-substituiertes 7-[4-(substituiertes)benzyl]amino-3-isopropylpyrazolo[4,3-d]pyrimidin der allgemeinen Formel I gemäß einer beliebigen der Ansprüche 1 bis 3 und mindestens einen pharmazeutisch verträglichen Träger enthält.

## Revendications

1. 5-Substitué-7-[4-(substitué)benzyl]amino-3-isopropylpyrazolo[4,3-d]pyrimidines de formule générale I 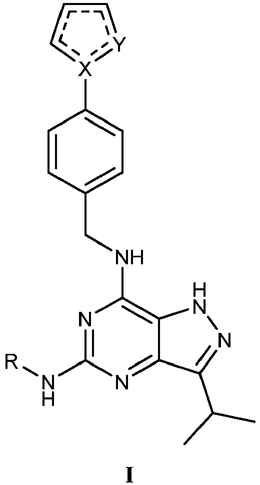 où
X est du carbone ou de l'azote et Y est CH, O, S ou N, les liaisons représentées par des lignes en pointillés (---) sont indépendamment des liaisons simples ou doubles;
R est choisi dans le groupe constitué de
- alkyle en C₂-C₁₀ linéaire ou ramifié, éventuellement substitué par au moins un substituant choisi parmi les substituants hydroxy et amino, de préférence par un substituant hydroxy et/ou un substituant amino;
- un groupe (dialkylamino)alkyle dans lequel chaque alkyle est choisi indépendamment parmi un alkyle linéaire ou ramifié en C₁-C₁₀;
- cycloalkyle C₃-C₁₀, qui est un groupe alkyle cyclique ou polycyclique contenant 3 à 10 atomes de carbone, éventuellement substitué par au moins un substituant choisi dans le groupe hydroxy, alkyle C₁-C₄, alcoxy C₁-C₄, hydroxyalkyle C₁-C₄ et amino substituants;
et leurs sels pharmaceutiquement acceptables, en particulier les sels avec les métaux alcalins, l'ammonium ou les amines, ou les sels d'addition avec les acides.

2. 5-Substitué-7-[4-(substitué)benzyl]amino-3-isopropylpyrazolo[4,3-d]pyrimidines de formule générale I selon la revendication 1, dans laquelle R est choisi dans le groupe comprenant l'éthyle, le propyle, butyle, 2-hydroxyéthyle, 3-hydroxypropyle, 2(R)-hydroxypropyle, 2(S)-hydroxypropyle, 4-hydroxybut-2(R)-yle, 4-hydroxybut-2(S)-yle, 4-hydroxybut-2(R,S)-yle, 2-(hydroxy-2-méthyl)propyle, 2,3-dihydroxypropyle, 1-hydroxy-3-méthylbutyle, (R,S)-2-hydroxypent-3-yle, (R)-2-hydroxypent-3-yle, (S)-2-hydroxypent-3-yle, (R)-1-isopropyl-2-hydroxyéthyle, (S)-1-isopropyl-2-hydroxyéthyle, 2-aminoéthyle, 3-aminopropyle, 4-aminobutyle, 5-aminopentyle, 6-aminohexyle, 3-amino-2-hydroxypropyle, 1-(diméthylamino)méthyle, 2-(diméthylamino)éthyle, 3-(diméthylamino)propyle, 4-(diméthylamino)butyle, 2-(diéthylamino)éthyle, 3-(diéthylamino)propyle, *cis*-2-aminocyclohexyle, *trans*-2-aminocyclohexyle, *cis,trans*-2-aminocyclohexyle, *cis,trans*-3-aminocyclohexyle, *trans*-4-aminocyclohexyle, *cis*-4-aminocyclohexyle, *cis,trans*-4-aminocyclohexyle, *cis*-2-hydroxycyclohexyle, *trans*-2-hydroxycyclohexyle, *cis,trans*-2-hydroxycyclohexyle, *cis,trans*-3-hydroxycyclohexyle, *trans*-4-hydroxycyclohexyle, *cis*-4-hydroxycyclohexyle, *cis,trans*-4-hydroxycyclohexyle.

3. 5-Substitué-7-[4-(substitué)benzyl]amino-3-isopropylpyrazolo[4,3-d]pyrimidines de formule générale I selon l'une quelconque des revendications 1 ou 2, dans lesquelles la structure 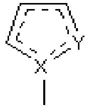 est choisie parmi le groupe contenant pyrrolidin-1-yle, 1H-pyrazol-1-yle, 1H-pyrrol-2-yle, thiophène-2-yle, furanne-2-yle.

4. 5-Substitué-7-[4-(substitué)benzyl]amino-3-isopropylpyrazolo[4,3-d]pyrimidines de formule générale I selon l'une quelconque des revendications précédentes pour utilisation comme médicaments.

5. 5-Substitué-7-[4-(substitué)benzyl]amino-3-isopropylpyrazolo[4,3-d]pyrimidines de formule générale I selon l'une quelconque des revendications 1 à 3 pour utilisation dans le traitement des maladies inflammatoires.

6. 5-Substitué-7-[4-(substitué)benzyl]amino-3-isopropylpyrazolo[4,3-d]pyrimidines de formule générale I selon l'une quelconque des revendications 1 à 3 pour utilisation dans le traitement de la polyarthrite rhumatoïde.

7. Composition pharmaceutique, **caractérisée en ce qu'**elle comprend au moins une 5-substitué-7-[4-(substitué)benzyl]amino-3-isopropylpyrazolo[4,3-d]pyrimidine de formule générale I selon l'une quelconque des revendications 1 à 3, et au moins un support pharmaceutiquement acceptable.
